# EUROPEAN PATENT SPECIFICATION

(11) **EP 1 143 994 B1**
(45) Date of publication and mention of the grant of the patent: **02.07.2003**
(21) Application number: 00900970.5
(22) Date of filing: 10.01.2000
(51) Int. Cl.: A61K 38/16, A61K 48/00, G01N 33/50, A61P 29/00, A61P 37/00

(54) **USE OF APOPTOSIS INDUCING AGENTS IN THE PREPARATION OF A MEDICAMENT FOR THE TREATMENT OF (AUTO)IMMUNE DISEASES**
VERWENDUNG VON APOPTOSE-INDUZIERENDE MITTEL ZUR HERSTELLUNG EINES MITTELS ZUR BEHANDLUNG VON (AUTO)IMMUNKRANKHEITEN
UTILISATION D'AGENTS INDUISANT L'APOPTOSE, DANS LA FABRICATION D'UN MEDICAMENT POUR LE TRAITEMENT DE MALADIES (AUTO)IMMUNES

(30) Priority: 11.01.1999 EP 99200056
(43) Date of publication of application: 17.10.2001
(73) Proprietor: Leadd B.V., 2333 AL Leiden (NL)
(72) Inventor: NOTEBORN, Mathieu, Hubertus, Maria, NL-2352 EH Leiderdorp (NL); PIETERSEN, Alexandra, Maria, NL-2312 GB Leiden (NL)
(74) Representative: Prins, Adrianus Willem
(86) International application number: NL0000013
(87) International publication number: WO00041497

(56) References cited:
- EP-A- 0 866 131
- WO-A-96/41191
- WO-A-98/37901
- WO-A-98/46760
- DE-A- 19 704 979
- DATABASE MEDLINE [Online] FILE SERVER STN KARLSRUHE; ABSTRACT NO. 1998352185, "Apoptin specifically causes apoptosis in tumor cells and after UV-treatment in untransformed cells from cancer-prone individuals: a review." XP002107637 & MUTATION RESEARCH, (1998 MAY 25) 400 (1-2) 447-55. REF: 52 JOURNAL CODE: NNA. ISSN: 0027-5107.,

## Description

The present invention relates to the field of therapies based on molecular biology. The invention further relates to diagnosis of the risk of future disease. More in particular the invention relates to the field of treatment and risk diagnosis of (auto)immune diseases and/or inflammatory disorders. The invention also relates to induction of apoptosis in certain cells associated with or related to (auto)immune diseases.

Autoimmune diseases are a group of severe diseases which are characterized by inflammatory disorders, such as Crohn's disease, chronic pancreatitis, some forms of diabetes, ulcerative colitis and rheumatoid arthritis (Bischoff et al., 1996; Firestein, 1995, 1998; Liblau et al., 1995). As one of the main representatives of this family of diseases, we will describe Rheumatoid arthritis (RA) in greater detail as representative of the applications of the present invention.

RA affects the joints but also other organs. The disease affects 1-2% of the adult population worldwide. Women are more frequently affected than men in a sex ratio of 3:1. The clinical spectrum as well as the course of the disease varies considerably. In a mild disease the joint inflammation may be present for a limited period of time and the joint destruction may not occur. This pattern is relatively rare. Most patients have continuously high or varying levels of disease activity. This is associated with a worse outcome of the disease with respect to disability and joint destruction (Van Zeben et al., 1994).

RA is associated with an increased risk of mortality (Wolfe, 1990). Joint destruction starts early in RA. The highest rate of erosion formation seems to be during the first 2 years after RA onset. Recently, evidence was provided that the following years a continuous progression of erosions will take place (Sharp et al., 1991; Van der Heijde et al., 1992).

The etiology of RA remains unresolved, although the pathophysiology of RA is a dynamic area of research (Breedveld, 1998). A simple scheme explaining this dramatic disease is that the inflammation and tissue destruction is initiated by the influx of lymphocytes into the synovium. They stimulate plasma cells, mast cells, macrophages and especially fibroblast-like synoviocytes to produce inflammatory mediators such as tumor necrosis factor-alpha and interleukin 1. These mediators can induce matrix degrading activities that eventually lead to joint destruction. These activities include the activation of fibroblast-like synoviocytes to produce collagenase, the induction of bone and cartilage resorption, and the increased expression of chemokines and of adhesion and HLA molecules, all of which lead to further stimulation of the immune response or to further influx of cells into the joint space (Breedveld, 1998).

Recently, data has been provided that FLS are irreversibly altered in RA and that an autonomous process allows them to remain activated even after removal from the articular inflammatory milieu. The cells continue to migrate and invade without additional exogenous stimulation, although reduced in comparison to the stimulated situation (Firestein, 1995). While cell division is one possible mechanism of FLS accumulation, evidence of profligate cell division and DNA synthesis in the intimal lining is scant. The present invention discloses that inducing apoptosis in these cells is useful in combating the effects of the disease.

If proliferation is, in fact, relatively low, then abnormalities in the rate of cell death may contribute to lining hyperplasia in synovitis. The extent of apoptosis in rheumatoid synovium has only recently been examined (Firestein et al., 1995, 1995a). Apoptosis is characterized by shrinkage of cells, segmentation by shrinkage of cells, segmentation of the nucleus, condensation and cleavage of DNA into domain-sized fragments, in most cells followed by internucleosomal degradation. Finally, the apoptotic cells fragment into membrane-enclosed apoptotic bodies, which are rapidly phagocytosed by neighboring cells. Therefore, apoptosis causes much less destruction of tissue than necrosis, the non-physiological type of cell death. (Wyllie et al., 1980; Arends and Wyllie, 1991).

Although, the mechanism of abnormal reduced apoptosis in RA has not been fully elucidated, a prominent role of defective p53 function seems to be involved with synoviocyte survival and death (Conway et al., 1995).
Mountz et al. (1994) have reported that defective apoptosis is related with other autoimmune diseases such as systemic lupus erythematous, vasculitis syndromes,
Behcet's diseases, and inflammatory bowel disease. Therefore, accorsing to the present invention a therapeutic approach for curing RA and other (auto) immune diseases will be to circumvent the apoptotic block in such cells by inducing an (alternative) apoptotic pathway. The invention therefor provides use of an apoptosis inducing agent, which exhibit its effect in aberrant cell, ivolved with or related to (auto) immune diseases in the preparation of a medicament for the treatment of inflammatory disorders and/or immune diseases wherein said apoptosis inducing agent apoptosis inducing protein apoptin or other proteins with apoptin-like activity. In particular the invention provides the use of an apoptosis inducing agent in the preparation of a medicament for the treatment of autoimmune diseases. The damage in all of the disorders/diseases mentioned above usually involves damage caused directly or indirectly by a certain subset of cells (such as FLS in RA) which are in some way out of control (excessive proliferation or other activity, or lack of regulated cell death, or necrosis or the like). It is therefor useful to be able to induce apoptosis in such a subset of cells by providing such cells with an apoptosis inducing agent.
Apoptosis is preferable to necrotic cell death, because it leads to less breakdown products, see above. Any manner in which the target cells can be provided with apoptotic activity is useful according to the present invention. It is however preferred that the apoptotic activity is provided by a proteinaceous substance which is encoded by a gene, delivered to the target cell by a gene delivery vehicle. A gene delivery vehicle is defined herein as any vehicle capable of delivering a gene to a cell, be it of viral or non-viral origin. The gene should be delivered in such a manner that it can be functionally expressed in the target cell.

The pharmaceutical formulation of the apoptosis inducing agent or the gene delivery vehicle will be similar to pharmaceutical formulations for other agents for inducing cell death for a certain population of target cells. For gene delivery vehicles, such as adenoviruses many formulations for delivering genes to certain numbers of cells have already been disclosed by many others and therefor such formulations need no further elaboration here. Other formulations for other gene delivery vehicles can be put together analogously or as described in the relevant art.

In order to be able to switch off any unwanted effects of the gene delivery vehicles according to the invention, it is preferred to add a suicide gene to its genetic information. Thus the invention also provides a use wherein said gene delivery vehicle further comprises a suicide gene. It is of course preferred that said gene is under control of an inducible promoter. Known suicide genes include genes encoding thymidine kinases or other cytotoxic proteinaceous substances.

In order to further reduce unwanted effects of the gene delivery vehicles according to the invention it is preferred that the gene delivery vehicle has (or is provided with) a tropism for its target cells, meaning that it has a higher binding and/or entering affinity for the target cells than for other cells. This can simply be achieved by selecting a gene delivery vehicle that has such a tropism, or by providing a delivery vehicle with such a tropism from an organism or substance that has an enhanced affinity for the target cell. If none such an organism or substance is available, it can be provided through phage display screening of random sequences having affinity for the target cells or other screening techniques. If a gene delivery vehicle is provided with tropism for a different target cell than its original tropism, this is often referred to as targeted gene therapy. Thus the invention also provides
a use according to the invention, wherein said gene delivery vehicle has a tropism for hematopoietic cells, or preferably for fibroblast-like synoviocytes. In the alternative the invention provides a use according to the invention wherein said gene delivery vehicle has been provided with a targeting means, especially a targetting means for fibroblast-like synoviocytes. A preferred gene delivery vehicle according to the invention is a recombinant adenovirus. Recombinant adenoviruses are well known in the field of gene therapy anhd need no further elaboration here. Safe ways to produce and use them have been disclosed in numerous publications.

The preferred apoptosis inducing agent according to the invention is apoptin or a functional fragment, derivative or equivalent thereof (proteins with apoptin-like activity). Apoptin is a protein derived from a chicken anemia virus and will be discussed in more detail below. A functional derivative includes a protein in which a number of amino acid residues have been modified or added without affecting the activity (meaning that it still induces apoptosis, albeit to another extent (higher or lower)). The same of course goes for fragments or combinations of fragments with derivatizations. Functional equivalents are counterparts of apoptin (chicken anemia virus protein 3) in other organisms. A very important advantage of apoptin over other apoptosis inducing agents is that it does not display its activity to any significant extent in normal cells, whereas the present invention shows that it does exhibit its effect in the aberrant cells involved with or related to (auto) immune diseases.

It is also preferred that apoptin expression be made inducible.

The invention further provides a test for the likelihood of cells to become aberrant in the manner of (auto) immune diseases. This test involves providing a sample of cells suspected of being able to become aberrant with a protein having apoptotic activity, such as a gene encoding apoptin or a derivative or fragment thereof, and thereafter subjecting said cells to stress, such as osmotic stress, heat shock, infectious stress, UV, etc. Cells which are aberrancy prone, will go into apoptosis following this treatment. Cells not having that potency will be mostly unaffected. Thus the likelihood of an individual for future (auto) immune diseases can be examined.

### Detailed description.

*In vitro*, synthesis of the chicken anemia virus (CAV)-derived protein apoptin, in chicken transformed cells, results in induction of apoptosis (Noteborn et al., 1994; Noteborn and Koch, 1995; Noteborn et al., Noteborn and Van der Eb, 1998). Apoptin is a small protein, only 121 amino acids long, which is rather basic, and rich with prolines, serines and threonines (Noteborn et al., 1991).

Apoptin, and other proteins with apoptin-like activity, can also induce apoptosis in human malignant and transformed cell lines, but not in non-transformed human cells (Danen-Van Oorschot et al., 1997; Noteborn et al., 1998a). We have established that apoptin-induced apoptosis occurs in the absence of functional p53 (Zhuang et al., 1995a), and cannot be blocked by Bcl-2, Bcr-Abl (Zhuang et al., 1995; 1995b), the Bcl-2-associating protein BAG-1 and the cow-pox protein CrmA (Noteborn, 1996; Danen-Van Oorschot et al., 1997a; Danen-Van Oorschot et al., 1998).
*In vitro*, apoptin fails to induce apoptosis in normal diploid lymphoid, dermal, epidermal, endothelial, or smooth muscle cells. However, when normal cells co-express apoptin and a transforming protein, such as SV40 Large T antigen, the cells will undergo apoptosis. These data indicate that apoptin-induced apoptosis will also take place under non-established tumorigenic situations (Noteborn et al., 1998b). In the analyzed transformed cells, which all undergo apoptin-induced apoptosis, apoptin is located within the cellular nucleus (Noteborn et al., 1998). In contrast, Apoptin was found predominantly in the cytoplasm of normal non-transformed cells (Danen-van Oorschot, 1997). However, co-expression with a transforming protein enables apoptin to be present in the nucleus, resulting in the induction of apoptosis (Noteborn et al., 1998a). Apoptin does not induce apoptosis, and is not localized in the nucleus of fibroblasts derived from cancer-prone individuals. However, after UV-irradiation (causing an aberrant SOS response in these cells resembling a transient transforming state) apoptin can induce apoptosis in these cells (Zhang et al, 1999). On the other hand, fibroblasts from healthy individuals did not respond to apoptin-induced apoptosis upon UV treatment. This illustrates that a predisposition is necessary, which upon induction will activate apoptin.

Recently, Noteborn and Pietersen (1998) have described the generation and characterization of an apoptin-expressing adenoviral vector AdMLPvp3. This vector allows an efficient synthesis of apoptin *in vitro* as well as *in vivo*. They demonstrated that Apoptin maintains its specificity for tumorigenic/transformed cells when introduced and expressed by an adenoviral vector. Experiments in rats demonstrated that AdMLPvp3 could be safely administered by e.g. intravenous injection. Repeated intravenous doses of AdMLPvp3 were also well tolerated, indicating that the apoptin-expressing virus can be administered without severe adverse effects. These results are strengthened by the fact that transgenic mice were generated, which produce apoptin in a large number of their cells (Noteborn and Zhang, 1998). A single intratumoral injection of AdMLPvp3 into a xenogeneic tumor resulted in a significant reduction of tumor growth (Pietersen et al., 1999).
The intrinsic specificity and the inherent low toxicity make apoptin-synthesizing adenovirus vectors promising tools for the treatment of solid tumors.

The invention in one embodiment now provides a gene therapy, which enables using the features of the apoptosis-inducing protein apoptin, or other proteins with apoptin-like activity for the manufacture of a medicament for treatment of autoimmune diseases, such as RA.

Such a gene delivery vehicle, which is an independently infectious vector; for example a virus or virus-derived vector, a liposome or a polymer, or the like, that in itself can infect or in any other way deliver genetic information to for example cells causing or involved in autoimmune diseases. The generic information comprises a nucleic acid molecule encoding a protein having apoptin-like activity. The invention also provides a gene delivery vehicle that greatly has been increased in its capacity to express apoptin-like apoptotic activity.

The gene delivery vehicle thus provided by the invention can for instance be an adenovirus, or a retrovirus, parvovirus or other DNA or RNA recombinant viruses that can be used as delivery vehicle or a plasmovirus. Additionally, the invention provides a gene delivery vehicle, which has also been supplemented with a specific ligand or target molecule or target molecules, by which the gene delivery vehicle can be specifically directed to deliver its genetic information at a target cell of choice. Such a target molecule can for instance be a viral spike protein, receptor molecule, or antibody reactive with a surface receptor or protein of cells related to autoimmune diseases.

Also, the invention provides a gene delivery vehicle, which can be used in the diagnosis i.e. autoimmune diseases, such as RA. Such a gene delivery vehicle can i.e. be used for *in vitro* diagnosis, wherein tissue or cell samples or biopsies are to be taken from a human or animal. Such samples can then be evaluated or tested by infecting them, in culture or directly, with said gene delivery vehicle capable of expressing i.e. apoptin-like activity. RA-related cells, such as fibroblast-like synoviocytes, or cells related to other autoimmune diseases will undergo apoptosis upon apoptin synthesis. Especially, when these cells are stimulated with growth, serum, cytokine factors and/or other factors inducing even more 'ggressive growth' of these cells. Alternatively, the nuclear location of apoptin in cells related with autoimmune diseases is another marker for diagnostic analysis of RA cells or cells, which are derived from other autoimmune diseases. The presence of apoptin can i.e. be demonstrated with classical (immuno) histochemical techniques i.e. microscopically or with automated cell sorting techniques.

In particular, the invention relates to anti-autoimmune therapies. Treatment of cells related with autoimmune diseases will take place by e.g. expression of apoptin by means of direct infection of these cells with gene delivery vehicles such as adenovirus vectors that contain a coding sequence for a protein with apoptin-like activity. Therefore, the invention in yet another embodiment provides gene delivery vehicles such as the adenovirus vector expressing apoptin, which is a potent anti-autoimmune agent. In addition, apoptin-expression in cells related with autoimmune disease will also indirectly cause cell death in those autoimmune-disease-related cells, which are not expressing apoptin. This so-called by-stander effect will improve the apoptin treatment of autoimmune diseases even more.

Apoptin synthesis does not or at least not detectably or significantly induce apoptosis in normal healthy cells, indicating that the toxicity of *in vivo* treatment with recombinant-apoptin vehicles, such as the adenovirus vector regulating the apoptin synthesis, is low.

Expression of apoptin in cells, which are related to autoimmune diseases may also take place by infecting cells with other DNA and/or RNA-viral vectors, besides adenovirus vectors, that contain a coding sequence for apoptin, such as retroviruses or parvoviruses (Lopez-Guerro et al., 1997). In addition, virus-derived vector systems, such as plasmoviruses (Noguiez-Hellin, 1996) can be used for the induction of apoptin-induced apoptosis in autoimmune-disease-related cells.

The invention also enables the identification of the essential cellular factors playing a role in the development of autoimmune diseases such as RA.

### Diagnostic assay for (auto)immune proneness

The data presented in this report allow us to develop an assay to determine whether an individual with an unknown cellular/genetic background, is prone for (auto)immune diseases compared to normal healthy persons. Normal diploid cells (such as FLS) from a (auto)immune-prone individual are insensitive to apoptin-induced apoptosis, but become so after stress-treatment, such as chemical, osmotic, heat, infectious, and/or irradiation, like UV- and X-rays. Below, an example of such a diagnostic assay is described based on the effect of an UV-irradiation.

Primary cells (e.g. FLS) are to be isolated from the individual to be tested and cultured in a suitable medium. Next, the cells are irradiated with UV and subsequently transfected with a plasmid encoding apoptin, or the cells are first transfected/infected and then irradiated. In parrallel, diploid cells from a normal healthy individual will be used as control.

By using an indirect immunofluorescence assay based on apoptin-specific Mab's, the cells are analysed for the presence of apoptin in the nucleus and/or for undergoing apoptosis. If the percentage of cells undergoing apoptosis among the apoptin-positive UV-treated cells is significantly higher than the percentage of apoptosis in UV-treated cells of a normal individual, this will be strong evidence that the individual from whom the cells are isolated, will be prone for (auto)immune diseases.

The invention will be explained in more detail on the basis of the following experimental part. This is only for the purpose of illustration and should not be interpreted as a limitation of the scope of the protection.

### EXPERIMENTAL PART

### Cells and cell culture conditions

Ad5 E1-transformed human embryonic retina (HER) PER.C6 cell lines were grown in Dulbecco's modified Eagle medium (DMEM) supplemented with 10% fetal calf serum (FCS) in a 5% C02 atmosphere at 370 C. Cell line PER.C6 was obtained from Fallaux et al. (1996). Cell culture media, reagents, and sera were purchased from GIBCO Laboratories (Grand Island, NY). Culture plastics were purchased from Greiner (Nurtingen, Germany).

Synoviocytes were derived from a patient suffering rheumatoid arthritis (RA). The cells were cultured in MDM medium containing 10% FCS. After adenoviral infection, the synoviocytes were cultured in MDM medium supplemented with 10% FCS or with 40% normal human serum. The synoviocytes were obtained from P. Goossens, Department of Rheumatology, Leiden University Medical Centre (LUMC), Leiden, The Netherlands.

### Viruses

The recombinant adenoviral vector AdMLPvp3 was used for the viral expression of apoptin (Pietersen et al., 1999). The vector AdMLPvp3 contains the E1A enhancer linked to the adenovirus Major Late promoter (MLP) to drive the apoptin gene, which comprises the chicken anemia virus (CAV)-derived region (positions nt 427-868; Noteborn et al., 1991). The recombinant adenoviral AdCMVLacZ was used as control adenovirus. AdCMVLacZ carries the E.coli LacZ gene for beta-galactosidase under the control of the Cytomegalovirus enhancer/promoter (Pietersen et al., 1999).

### Virus techniques

Plaque assays were performed, as described previously (Graham and Prevec, 1991). Briefly, adenovirus stocks were serially diluted in 2 ml DMEM containing 2% horse serum and added to near-confluent PER.C6 in 6-well plates. After 2h incubation at 370 C, the medium was replaced by F-15 minimum essential medium (MEM) containing 0.85% agarose (Sigma, USA), 20 mM HEPES (pH 7.4), 12.3 mM MgCl2, 0.0025% L-glutamine, and 2% horse serum (heat-inactivated at 560 C for 30 minutes).

Small-scale production of adenovirus lots was performed as described by Fallaux (1996). Briefly, near-confluent PER.C6 monolayers were infected with approximately 5 plaque-forming units (pfu's) per cell, in phosphate-buffered saline (PBS) containing 1% horse serum. After 1 hour at room temperature, the inoculum was replaced by fresh medium (DMEM/2% horse serum). After 48 hours, the nearly completely detached cells were harvested, and collected in 1 ml PBS/1% horse serum. Virus was isolated from the producer cells by 3 cycles of flash-freeze/thawing. The lysates were cleared by centrifugation at 3000 rpm for 10 minutes, and stored at -20° C.

The PER.C6 produced rAdV stocks were screened for the presence of recombinant-competent adenovirus by performing PCR analysis with primers derived from the Ad5 ITR region (5'-GGGTGGAGTTTGTGACGTG-3') and the E1A encoding region (5'-TCGTGAAGGGTAGGTGGTTC-3') as described by Noteborn and De Boer (1995) using a Perkin Elmer PCR apparatus. The presence of a 600-bp amplified fragment indicates that replication-competent (E1-region containing) adenovirus exists in the analysed virus stock (Pietersen et al., 1999).

### Immunofluorescence and DAPI staining

Indirect immunofluorescence was performed as described by Noteborn et al. (1990). To demonstrate the presence of Apoptin and to establish its cellular localization in infected cells, the cells were fixed with 80% acetone. The indirect immunofluorescence assay was performed with a 3-fold dilution of the mouse monoclonal antibody (mAb) CVI-CAV-111.3 for Apoptin and a 100-fold dilution of mAb LacZ (Boehringer Mannheim, The Netherlands) for beta-galactosidase. Fluorescein-isothiocyanate-labeled goat anti-mouse antibody (Jackson ImmunoResearch Laboratories Inc., West Grove PA, USA) was used as second antibody. Nuclear DNA was stained with 1 microgram per milliliter 2,4-diamino-2-phenylindole (DAPI), 2% 1,4 diazabicyclo[2,2,2]-octane (DABCO) in glycerol/0.1 M TrisHCl pH 8.0 (Telford et al., 1992).

### TUNEL assay

Terminal-deoxynucleotidyl-transferase (Tdt)-mediated dUTP nick end labeling (TUNEL) was performed with the use of the in-situ cell death detection kit(Boehringer Mannheim, Germany). Twenty-four hours after infection, cells were washed with PBS and fixed with 4% paraformaldehyde in PBS (pH 7.4) for 30 minutes at room temperature. After permeabilisation (0.1% Triton X-100, 0.1% sodiumcitrate, 2 minutes at 4° C) cells were incubated with the TUNEL reaction mixture (containing fluorescein-labelled nucleotide polymers and terminal-deoxynucleotidyl transferase) for 1 hour at 37° C. After washing with PBS, the cells were analysed by fluorescence microscopy.

### Giemsa staining and beta-galactosidase assays

For detection of the number of attached cells, cells were stained with Giemsa. After (mock)infection, the cells were washed twice with PBS and fixed in methanol:acetic acid (3:1) for 15 minutes at room temperature. For 30 minutes, cells were incubated in a 3% Giemsa solution (Merck, Darmstad, Germany) in 1 mM Na2HPO4, pH 7.0) at room temperature. After staining, the cells were washed 4 times with deionized water and allowed to dry by air.

For detection of LacZ-encoded beta-galactosidase activity, cells in tissue culture were fixed 24 hours after infection in ice-cold 2% paraformaldehyde/0.2% glutaraldehyde solution, washed in ice-cold PBS (containing 2 mM MgCl2), and incubated in 3 ml of reaction mix (1 milligram per milliliter X-gal (Boehringer Mannheim, Germany), 5 mM potassium ferrocyanide, 5 mM potassium ferricyanide, 2 mM MgCl2 in PBS) at 37° C for 4-16 hours (Sanes et al., 1986).

### RESULTS AND DISCUSSION

### An in vitro model for the human autoimmune disease rheumatoid arthritis (RA).

To study possible therapeutic effects of synthesis of apoptin for RA patients, we have established an *in vitro* model for RA. To that end, fibroblast-like synoviocytes (FLS) from patient OH. suffering RA were isolated. The cells are cultured in 'non-stimulating' medium containing fetal calf serum or in so-called 'stimulating' medium, which contains 40% normal human serum. Especially, the latter medium contains cytokines and other stimulating factors, which closely resemble the RA situation *in vivo.*
These 'stimulated' LFS mimic the RA conditions concerning another very important aspect. The aberrant growth of LFS *in vivo* and *in vitro* will cause secretion of various cellular factors stimulating their own cell growth and those of others (e.g. LFS) even more (Firestein, 1995). However, the (cultured) RA-related LFS have also undergone intrinsic genetic changes, which already makes them already different in comparison to normal healthy cells.

### Adenovirus vectors are very suitable for expression of a transgene in RA-related LFS.

At present the most efficient system to achieve the transduction of a transgene for the majority of cell types makes use of adenoviral vectors. These vectors have several advantages that make them particularly suitable for *in vivo* gene transfer. Recombinant adenoviral vectors can be grown to high titers, have the capacity to transduce non-mitotic cells, and do not integrate their genomes into host-cell DNA. Moreover, adenovirus vectors have already been applied for clinical gene-therapy trials.

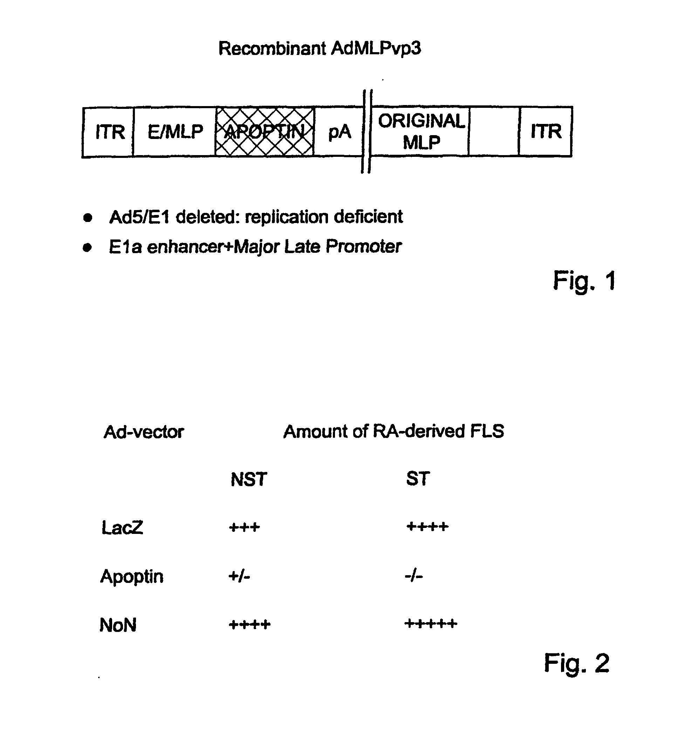

We have examined whether a recombinant-apoptin adenovirus vector might result in an efficient transduction of RA LFS cells. To that end, these LFS cells were infected with the replication-deficient AdLacZ vector (moi 50), which encodes the beta-galactosidase protein. Two days after infection, the LFS cells were fixed and analysed for beta-galactosidase synthesis. Approximately, 40% of the infected FLS were positive in the beta-galactosidase assay. In comparison to other cell types infected with recombinant adenovirus vectors expressing beta-galactosidase, this transduction percentage is high.
Therefore, we conclude that an adenovirus vector is very suitable for producing transgenes in RA FLS. An example of an adenovirus vector suitable for the expression of apoptin is shown in Figure 1.

### Infection of serum-stimulated RA LFS with AdMLP-vp3 results in a dramatic level of cell death.

Next, we have determined the cytotoxic effect of apoptin synthesis in RA LFS. To that end, the cells were infected with the negative control virus AdLacZ, AdMLPvp3 (both moi: 50) encoding apoptin or mock-infected. Subsequently, the cells were grown in 'non-stimulating' or 'stimulating' medium. Three and six days after infection, the cells were analysed for cell density by Giemsa staining.

Three days and six days after infection, the cells that were infected with recombinant adenovirus vector AdLacZ did not show a significant reduction in cell density, in comparison with those that were mock-treated. These data were observed for both the 'non-stimulating' as well as the 'stimulating' medium conditions.

On the other hand, the cell density in the dishes with RA FLS that were infected with the recombinant vector AdMLPvp3, however, was significantly reduced. Already, three days after infection (two days after 'stimulation') the RA FLS almost all 'died'. The dishes that were not stimulated did not seem to have a significant reduction of cells caused by infection with AdMLP-vp3. However, six days after infection, the amount of AdMLPvp3-treated cells was also significantly reduced compared with mock-treated RA FLS. The results of the experiments, showing the effect on the cell density of RA FLS cultures infected with AdLacZ, AdMLPvp3 or mock-treated at six days after infection, are shown in Figure 2.

These results prove that apoptin synthesis specifically causes cell death in FLS, which are derived from a patient suffering the autoimmune disease RA. The infection of adenovirus vectors, as such, has no significant cytotoxic effect on RA FLS. Apoptin already has a moderate negative effect on the cell density of RA FLS, when they are grown under 'non-stimulating' conditions. These data imply, that the RA FLS are different from human normal healthy cells, as has been suggested by Firestein (1995, 1997, 1998) and others (Breedveld, 1997). This difference seems to be 'recognized' by apoptin.

The fact that apoptin has a more potent cell-killing effect when the RA FLS are serum-stimulated, indicates that apoptin becomes even more activated when the FLS start secreting various factors such as cytokines, chemokines, etc., which all have an enhancing effect on the cell proliferation (Firestein, 1997) leading to a more transformed-like status.

The results are even more interesting when one takes into account that AdMLP-vp3 infection causes apoptin production in approximately 30-40% of the RA-derived FLS (as determined by immunofluorescence analysis in parallel infected FLS cultures), whereas almost all FLS are killed. Not only the apoptin-positive FLS, but also the apoptin-negative cells are killed. This result indicates that the AdMLPvp3 treatment has a by-stander effect. Most likely, a dramatic reduction of growth-stimulating and/or apoptosis-preventing factors, due to the apoptin-induced apoptosis will cause the death of the apoptin-negative cells too.

We conclude that apoptin can induce cell death in RA FLS, which is even enhanced by exogenous and endogenous factors. These features imply that apoptin will be a therapeutic agent for curing RA and other autoimmune diseases.

### TUNEL analysis proves that apoptin synthesis mediated by AdMLP induces apoptosis in RA LFS.

To characterize the nature of AdMLPvp3-induced cell death, we visualized the presence of DNA strand breaks with the aid of the enzyme terminal deoxynucleotidyl transferase and Fitc-labeled dUTP (TUNEL assay). RA FLS were infected either with AdMLP-vp3 or with AdLacZ and after 24 hours the cells were stimulated with 40% human serum. One day later, the cells were harvested and stained for the transgene to confirm similarity in transduction efficiencies and parallel-infected dishes were subjected to the TUNEL assay. Even though 40% of the RA FLS were expressing beta-galactosidase, only occasionally a single cell exhibited DNA breaks that could be detected by the TUNEL assay. In contrast, the frequency of TUNEL-positive cells after AdMLPvp3 infection appeared to be in the same range as the frequency of Apoptin-positive cells after infection.

Therefore, we conclude that apoptin can induce apoptosis in cells, which have lost or reduced their own potential to undergo apoptosis. The fact, that apoptin can induce apoptosis in these RA LFS cells, indicates 5 that apoptin treatment in vivo will cause a very low level of side effects such as inflammatory reactions.

### Nuclear localization of apoptin in human-serum-stimulated RA LFS.

To examine, the cellular localization of apoptin in 'stimulated' RA-derived FLS, the cells were infected with AdMLPvp3 for 1 day, serum-stimulated for an additional day and analysed by immunofluorescence using an apoptin-specific monoclonal antibody and DAPI-staining. Almost all apoptin-positive cells contained apoptin in the cellular nucleus. Already a high amount of apoptin-positive cells already contained aberrant bright DAPI structures, which are indicative of very late apoptotic conditions; namely condensed chromatin/DNA. These results again prove that apoptin synthesis results in the induction of apoptosis in RA FLS.
Thusfar, all apoptin-sensitive cells for cellular conditions showed a nuclear localization of apoptin (Noteborn et al., 1998b). The presented data proves that the apoptin activity in RA FLS is also correlated with its nuclear localization.

### Infection of serum-stimulated LFS derived from two other EA patients with AdMLP-vp3 results in a dramatic level of cell death.

To examine whether synthesis of apoptin results in induction of apoptosis in various RA patients, RA-related FLS have also been extracted from the RA patients designated 'E' and 'Lo'. The RA-related FLS derived from these RA patients have been obtained from the Department of Rheumatology, Leiden University Medical Center, Leiden, The Netherlands.

To that end, the LFS from the patients 'E' and 'Lo' were infected with AdMLPvp3 encoding apoptin, the negative control virus AdLacZ encoding the non-apoptotic protein beta-galactosidase (both moi: 50) or mock-infected. Subsequently, the cells were grown in 'non-stimulating' or 'stimulating' medium. Three days after infection (two days after serum stimulation), the cells were analyzed for cell density by DAPI staining (Noteborn et al., 1998b). The 'non-stimulated' as well as the 'stimulated' cells that were infected with recombinant adenovirus AdLacZ did not show a significant reduction in cell density, in comparison with those that were mock-treated.

In contrast, the cell density in the dishes with RA FLS derived from both patients 'E' and 'Lo' that were infected with the recombinant vector AdMLPvp3 was significantly less. Already, the AdMLPvp3-treated dishes that were not stimulated were less dense than the ones infected with AdLacZ or mock-treated. This effect was even more significant in the cases where the RA FLS had been 'stimulated' with 40% normal human serum.
The obtained results of these experiments and the one based on RA FLS derived from patient OH, prove that apoptin synthesis specifically causes cell death in FLS, which are derived from various patients suffering the autoimmune RA. Infection of adenovirus vectors, as such, has no significant negative effect on the cell density of RA FLS. These features strengthens the above described statement that apoptin is a therapeutic agent for curing RA and other autoimmune diseases.

### Construction of the adenovirus vector AdApt-Apoptin.

The adenovirus vector AdMLP-vp3 regulates the expression of the apoptin gene under the control of the adenovirus major late promoter (MLP). The novel AdApt adenoviral vector contains the cytomegalovirus (CMV) promoter, which has also been optimally adapted to the helper cell line PER.C6.

To examine whether it is possible to produce apoptin by means of a adenovirus vector under the regulation of the CMV, we have constructed AdApt-Apoptin.

To that end, the BamHI fragment from plasmid pCMV-vp3 (Noteborn, 1996) containing the Apoptin-encoding sequences (Noteborn et al., 1991) was cloned into the BamHI site of the 6.1-kb transfer vector AdApt, which was obtained from IntroGene, Leiden, The Netherlands. By sequence analysis and restriction-enzyme digestions the correct orientation of the apoptin gene under the regulation of the CMV was determined. This transfer vector has been named pAdApt-Apoptin. As negative control adenovirus transfer vector the plasmids were selected, which contain the apoptin gene in the wrong orientation opposite to the CMV promoter and is named AdApt-AS.
Next, recombinant adenovirus vectors expressing the apoptin gene under the regulation of the CMV promoter were generated. In addition, also control adenovirus harboring the apoptin gene in the opposite orientation relative to the CMV promoter was made. To that end, PER.C6 cells (IntroGene, Leiden, The Netherlands) were co-transfected with the adenovirus vector plasmid pAd5AlfII-ITR (E1-, E3+) and with the transfer plasmids pAdApt-Apoptin or pAdApt-AS. After the observation of cytopathogenic effects of the transfected PER.C6, the medium containing the recombinant adenovirus vectors were harvested and plaque-purified (Noteborn and Pietersen, 1998). The various plaque-purified recombinant adenovirus batches AdApt-Apoptin encoding the Apoptin and control vector AdApt-AS were examined by PCR-analysis for the presence of the Apoptin gene in the 'correct' versus 'wrong' orientation, respectively (Pietersen et al., 1999). All analyzed (in total for each vector type at least 10) recombinant adenovirus batches contained the expected apoptin gene. RCA analysis by means of PCR (Pietersen et al. 1999) revealed that in all analyzed batches no replication-competemt adenovirus was generated. Finally, the production of apoptin protein by AdApt-infected human HepG2 cells was examined by means of in-direct immunofluorescence using the monoclonal antibody 111.3 (Noteborn and Pietersen, 1998). The cells were almost all shown to produce apoptin protein and became very soon after infection apoptotic. This finding is indicative for the fact that the produced apoptin is completely active as an apoptotic inducer. As expected, all cells infected with AdApt-AS did not stain for the monoclonal antibody and did not become apoptotic. In conclusion, the fact that we are able to produce apoptin by means of various recombinant adenovirus vectors, either under the regulation of the adenovirus MLP or the CMV promoter, indicates that apoptin can be produced in any adenoviral vector without limiting the virus-vector production.
Infection of 'serum-stimulated' RA FLS, derived from the RA patient 'E', with AdApt-Apoptin resulted in a significant induction of cell death as has been described above for the AdMLP-vp3 recombinant adenovirus vector. Therefore, one can conclude that besides the recombinant adenovirus vector AdMLP-vp3 also other adenovirus vector expressing the apoptin gene such as the AdApt-Apoptin recombinant adenovirus can be used adenovirus vector as base for a therapy against autoimmune diseases such as RA.

### Diagnostic assay for auto-immune disease cells based on rAd-apoptin.

A marker for autoimmune-disease-related cells is the responsiveness to apoptin-induced apoptosis. Especially, upon stimulation of these cells with factors related to auto-immune diseases, such as certain cytokines and growth factors, will result in programmed cell death induced by synthesis of apoptin. Furthermore, another marker is the cellular localization of apoptin, which is different for apoptin-sensitive cells related to auto-immune disease in comparison to normal healthy cells.

By infecting cells with a vehicle expressing apoptin, such as a recombinant adenovirus regulating the synthesis of apoptin, and analyzing the apoptin cellular localization and/or induction of apoptosis within these cells, one is able to prove whether a cell is derived from a patient suffering an autoimmune disese or not. Especially, upon (serum)-stimulation the nuclear apoptin location and induction of apoptosis will increase significantly.

For instance, the cells are infected with an adenovirus expressing apoptin and in parallel with a control adenovirus, such as AdLacZ. The cells will be checked for apoptin in the cytoplasm or in the nucleus (autoimmune-related cells) by means of an e.g. immunofluorescence assay based on monoclonal antibodies specific for apoptin, such as 111.3 (Danen-Van Oorschot et al., 1998). In addition or instead of, the percentage of apoptotic cells will be estimated.
If the percentage of apoptotic cells is significantly higher for cells synthesizing apoptin in comparison to cells containing an exogenous control protein, such as beta-galactosidase, these cells are derived from patients suffering an autoimmune disease.

### Diagnostic assay for the identification of-factors causing auto-immune diseases

Besides the intrinsic changes of autoimmune disease cells, the secretion of various factors by these cells and most likely by other (immune) cells will increase the severeness of the autoimmune disease, such as RA.

Therefore, the above described diagnostic assay for the identification of cells related to autoimmune diseases, can also be used for the identification of factors, which cause and/or improve the 'aggressiveness' of cells causing clinical signs of RA or other auto-immune-diseases.
Upon treatment with such a factor, cells such as human (RA) fibroblast-like synoviocytes, will undergo extensive apoptin-induced apoptosis and/or harbor apoptin in their nucleus.

### Apoptin-induced apoptosis is indicative of transformed-like conditions within cells related to autoimmune diseases.

The fact that apoptin can induce apoptosis in ('stimulate') RA FLS, indicates that these cells are in a transformed condition. Thusfar, apoptin was proven not to induce apoptosis in normal non-transformed cells, which were from human or other mammalian origin (Danen-Van Oorschot et al., 1997, Noteborn et al., 1998b, Zhang et al., 1999). These data are strenghtened by the fact that transgenic mice; expressing apoptin in various of their tissues, are looking normal. None of their organs seems to undergo enhanced apoptotis, due to synthesis of apoptin in their cells (Noteborn and Erkeland, unpublished results).
UV-induction of aberrant stress-related processes in normal non-transformed cells, derived from individuals with cancer-prone syndromes, however, enables apoptin to induce apoptosis in these cells (Zhang et al., 1999) during a transient period. Apoptin does not induce apoptosis in UV-treated cells of healthy individuals. Apoptin can induce apoptosis rather moderately in RA FLS. For instance serum-stimulation of RA FLS increases the level of apoptin-induced apoptosis in i.e. RA FLS. It seems that these RA FLS are already different from normal healthy cells, but become even more aberrant
(transformed) after 'stimulation'. These features resemble those described for the UV-treated cells derived from cancer-prone individuals (Zhang et al., 1999). In both cases, a cellular process has been changed, which under 'normal' conditions can be handled by the cell, but upon specific stimuli will result in aberrant cellular processes leading to the accelerated development of cancer or autoimmune diseases.

Rheumatoid FLS often appear and behave like normal fibroblasts, which has led to the notion that they respond to their environment rather than act as independent aggressors (being transformed). However, some fragmentary evidence has been provided that they also exhibit characteristics of transformed cells. For instance, adherence to plastic or extra-cellular matrix is generally required for normal fibroblasts to proliferate and survive in culture for prolonged periods of time. Transformed cells, however, can grow in suspension in semi-solid medium without contact with a solid surface. While FLS typically grow and thrive under conditions that permit adherence, they can, in some circumstances, proliferate in an anchorage-independent manner (Lafyatis et al., 1989). Furthermore, the expression of several oncogenes such as c-myc has been reported for cultured FLS (Gay and Gay, 1989). Higher endogenous release of growth factors such as tumor growth factor-beta and other cytokines have also been described for FLS (Bucala et al., 1991; Remmers et al., 1990; Geiler, 1994; Firestein, 1995 and 1995a). Also, in some cases non-functional tumor-suppressor p53 has been related with RA (Aupperle et al., 1998). Although mutant p53 is not an oncogene, it prevents induction of apoptosis by endogenous or exogenous agents other than apoptin.

All these data indicate that FLS are irreversibly altered in RA and that an autonomous process allows them to remain activated even after removal from the articular inflammatory milieu (Firestein, 1995). We have provided evidence that apoptin can recognize these transformed-like autoimmune conditions, which enables the identification of the cellular factors being important in such diseases.

### DESCRIPTION OF THE FIGURES

Figure 1 shows the diagrammatic representation of the essential parts of the recombinant adenovirus AdMLP-vp3, which contains the gene encoding apoptin, under the regulation of the adenoviral major late promoter.
Figure 2 shows the schematic representation of the apoptin-induced cytotoxic effect in cultured fibroblast-like synoviocytes (FLS) derived from the synovium of a patient suffering from rheumatoid arthritis. 1.5 x 104 cells were cultured in 24-well dishes for 24 hours, infected with recombinant adenovirus AdLacZ expressing beta-galactosidase (LacZ), with AdMLP-vp3 encoding apoptin (Apoptin) or mock-infected (NON). The FLS were grown under normal conditions (NST) or 1 day after infection, stimulated with 40% normal human serum (ST) inducing more aggressively growing FLS, which resembles the RA situation *in vitro.* Finally, six days after infection with adenovirus virus or mock-infection, the cell monolayers were fixed and stained with GIEMSA solution. (+: represents an amount of living/attached cells; -: means no surviving/attached cells

### REFERENCES

1. Arends, M.J., and Wyllie, A.H. 1991. Apoptosis: mechanisms and roles in pathology. International Rview of Experimental Pathology 32, 223-254.
2. Aupperle, K.R., Boyle, D.L., Hendrix, M., Seftor, E.A., Zvaifler, N.J., Barbosa, M., and Firestein, G.S. Regulation of synoviocyte proliferation, apoptosis, and invasion by the p53 tumor-suppressor gene. (1998). American J. Pathology 152, 1091-1098.
3. Bischoff, J.R., Kirn, D.H., Williams, A., Heise, C., Horn, S., Muna, M., Ng, L., Nye, J., Sampson-Johannes, A., Fatteay, A., and McCormick. An adenovirus mutant that replicates selectively in p53-deficient human tumor cells. (1996). Science 274, 373-376.
4. Breedveld, F. New insights in the pathogenesis of Rheumatoid arthritis. (1998). The J. of Rheumatology 25, 3-7.
5. Bucala, R., Ritchlin, C., Winchester, R., Cerami, A. Constitutive production of inflammatory and mitogenic cytokines by rheumatoid synovial fibroblasts. (1991). J. Experimental Medicine 173, 569-574.
6. Conway, J.G., Wakefield, J.A., Brown, R.H., Marron, B.E., Sekut, L., Stimpson, S.A., McElroy, A., Menius, J.A., Jeffreys, J.J., Clark, R.L., McGeehan, G.M., and Conolly, K.M. Inhibition of cartilage and bone destruction in adjuvant arthritis in the rat by a matrix metalloproteinase inhibitor. (1995). J. Experimental Medicine 182, 449-457.
7. Danen-Van Oorschot, A.A.A.M., Den Hollander, A., Takayama, S., Reed, J.C., Van der Eb, A.J., and Noteborn, M.H.M. Bag-1 inhibits p53-induced apoptosis but not apoptin-induced apoptosis. (1997). Apoptosis 2, 395-402.
8. Danen-Van Oorschot A.A.A.M., Fischer D., Grimbergen J.M., Klein B., Zhuang S.-M., Falkenburg J.H.F., Backendorf C., Quax P.H.A., Van der Eb A.J., Noteborn M.H.M. Apoptin induces apoptosis in human transformed and malignant cells but not in normal cells. (1997a). Proceedings National Academy Sciences, USA 94: 5843-5847.
9. Danen-van Oorschot, A.A.A.M., van der Eb, A.J., and Noteborn, M.H.M. Apoptin-induced apoptosis: antitumor potential? (1998). In: Proceedings of the Royal Netherlands Academy of Arts and Sciences Pharmaceutical Intervention in apoptotic pathways, pp 199-212.
10. Gay, S. and Gay, R.E. Cellular basis and oncogene expression of rheumatoid joint destruction. (1989). Rheumatology International 9, 105-113.
11. Geiler, T., Kriegsmann, J., Keyzer, G.M., Gay, R.E., Gay, S. A new model for rheumatoid arthritis generated by engraftment of rheumatoid synovial tissue and normal human cartillage into SCID mice. (1994). Arthritis Rheumatology 37, 1664-1671.
12. Fallaux, F. (1996). Gene Therapy for haemophilia A: Towards the use of adenoviral vectors? PhD Thesis, Leiden University, The Netherlands.
13. Firestein, G.S. Invasive fibroblast-like synoviocytes in rheumatoid arthritis. (1995). Arthritis and Rheumatism 39, 1781-1790.
14. Firestein, G.S. Apoptosis in rheumatoid arthritis synovium. (1995a). J. Clinical Investigations 96, 1631-1638.
15. Firestein, G.S. Rheumatoid arthritis: Etiology and Pathogenesis of Rheumatoid arthritis. (1997). Textbook of Rheumatology, 5th edition. Eds. Kelley, Harris, Ruddy and Sledge. Chapter 54, pp 851-897.
16. Firestein, G.S., Novel therapeutic strategies involving animals, arthritis, and apoptosis. (1998). Current opinion in Rheumatology 10, 236-241.
17. Graham, F.L., and Prevec, L. (1991). Manipulation of adenovirus vectors. In: Methods in Molecular Biology. Volume 7: Gene Transfer and Expression Protocols. E.J. Murray, ed. The Humana Press, Clifton, N.J., USA, pp 109-128.
18. Lafyatis, R., Remmers, E.F., Roberts, A.B., Yocum, D.E., Sporn, M.B., and Wilder, R.L. Anchorage-independent growth of synoviocytes from arthritic and normal joints: stimulation by exogenous platelet-derived growth factor and inhibition of by transforming growth factor beta and retinoids. (1989). J. Clinical Investigations 83, 1267-1276.
19. Liblau, R.S., Singer, S.M., McDevitt, H.O. Th1 and Th2 CD4+ T cells in the pathogenesis of organ-specific autoimmune diseases.(1995). Immunol Today 16, 34-38.
20. Lopez-Guerro, J.A., Rayet, B., Tuynder, M., Rommelaere, J., and Dinsart, C. (1997). Constitutive activation of U937 promonocytic cell clones selected for their resistance to parvovirus H-1 infection. Blood 89, 1642-1653.
21. Mountz, J.D., Wu, J., Cheng, J., Zhou, T. Autoimmune disease: a problem of defective apoptosis. (1994). Arthritis and Rheumatism 37, 1415-1420.
22. Noguiez-Hellin, P., Robert-LeMeur, M., Salzmann, J.L., and Klatzmann, D. (1996). Plasmoviruses: nonviral/viral vectors for gene therapy. Proceedings National Academy Sciences USA 93, 4175-4180.
23. Noteborn, M.H.M. Apoptin induces apoptosis in human transformed and malignant cells but not in normal cells as essential characteristic for the development of a anti-tumor therapy. 1996. PCT application WO 96/41191.
24. Noteborn, M.H.M. and De Boer, G.F. (1995). Patent USA/no US 030, 335.
25. Noteborn, M.H.M., Danen-van Oorschot, A.A.A.M., and van der Eb, A.J. Chicken Anemia Virus: Induction of apoptosis by a single protein of a single-stranded DNA virus (1998). Seminars in Virology 8, 497-504.
26. Noteborn, M.H.M., Danen-van Oorschot, A.A.A.M., and van der Eb, A.J. The Apoptin® gene of chicken anemia virus in the induction of apoptosis in human tumorigenic cells and in gene therapy of cancer. (1998a). Gene Ther. Mol. Biol. Vol. 1, 399-406.
27. Noteborn, M.H.M., De Boer, G.F., Kant, A., Koch, G., Bos, J., Zantema, A., and Van der Eb, A.J. Expression of avian leukemia virus env-gp85 in Spodoptera frugiperda cells using a baculovirus expression vector. (1990). J. General Virology 71, 2641-2648.
28. Noteborn M.H.M., De Boer G.F., Van Roozelaar D.J., Karreman C., Kranenburg O., Vos J.G., Jeurissen S.H.M., Hoeben R.C., Zantema A., Koch G., Van Ormondt H., Van der Eb A.J. Characterization of cloned chicken anemia virus DNA that contains all elements for the infectious replication cycle. (1991) J Virology 65, 3131-3139.
29. Noteborn, M.H.M., Koch, G., Chicken anaemia virus infection: molecular basis of pathogenicity. Avian Pathol. 24 (1995), 11-31.
30. Noteborn, M.H.M. and Pietersen, A.M. A gene delivery vehicle expressing the apoptosis-inducing proteins VP2 and/or apoptin. 1998. PCT application no PCT/NL98/00213
31. Noteborn M.H.M., Todd D., Verschueren C.A.J., De Gauw H.W.F.M., Curran W.L., Veldkamp S., Douglas A.J., McNulty M.S., Van der Eb A.J., Koch G. A single chicken anemia virus protein induces apoptosis. (1994). J. Virology 68, 346-351. Noteborn, M.H.M. and van der Eb, A.J. Apoptin induced apoptosis: potential for antitumor therapy. (1998). Drug Resistance Updates 1, 99-103.
   Noteborn, M.H.M. and Zhang, Y.-H. Methods and means for determining the transforming capability of agents, for determining the predisposition of cells to become transformed and prophylactic treatment of cancer using apoptin-like activity. (1998). PCT application no PCT/NL/98/0725.
34. Noteborn, M.H.M., Zhang, Y.-H., and van der Eb, A.J. (1998b). Apoptin specifically causes apoptosis in tumor cells and after UV-treatment in untransformed cells from cancer-prone individuals: A review, Mutation Research 400, 447-455.
35. Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, A.J., Hoeben, R.C., and Noteborn, M.H.M. Specific tumor-cell killing with adenovirus vectors containing the Apoptin gene (1999). Gene Therapy, in press.
36. Remmers, E.F., Lafyatis, R., Kumkumian, G.K., Case, J.P., Roberts, A.B., Sporn, M.B., and Wilder, R.L. Cytokines and growth regulation of synoviocytes from patients with rheumatoid arthritis and rats with streptococcal cell wall arthritis. (1990). Growth Factors 2, 179-188.
37. Sanes, J.R., Rubenstein, J.L., Nicolas, J.F. Use of 5 a recombinant retrovirus to study post-implementation cell lineage in mouse embryos. (1986). EMBO J. 5, 3133-3142.
38. Sharp, J.T., Wolfe, F., Mitchell, D.M., and Bloch, D.A. The progression of erosions and joint space narrowing scores in theumatoid arthritis during the first twenty-fiv years of disease. (1991). Arthritis and rheumatism 34, 660-668.
39. Telford, W.G., King, L.E., Fraker, P.J. Comparative evaluation of several DNA-binding dyes in the detection of apoptosis-associated chromatin degradation by flow cytometry. (1992). Cytometry 13, 137-143.
40. Van der Heyde, D.M., Van Leeuwen, M.A., Van Riel, P.L., Koster, A.M., Van 't Hof, M.A., Van Rijswijk, M.H., and Van der Putte, L.B. Biannual radiographic assessments of hands and feet in a three-year prospective following patients with early rheumatoid arthritis. (1992). Arthritis and Rheumatism 35, 26-34.
41. Van Zeben, D. Hazes, J.M., Zwinderman, A.H., Vandenbroucke, J.P., and Breedveld, F.C. The severity of rheumatoid arthritis: a 6-year follow-up study of younger women with symptoms of recent onset. (1994). J. Rheumatology 21, 1620-1625.
42. Wyllie, A.H., Kerr, J.F.R., Currie, A.R. (1980). Cell death: The significance of apoptosis. International Review of Cytology 68, 251-306.
43. Wolfe, F. 50 years of antirheumatic therapy: the prognosis of rheumatoid arthritis. (1990). J. Rheumatology 17 (suppl 22), 24-32.
44. Zhang, Y., Abrahams, P., van der Eb, A.J., Noteborn, M.H.M. (1999). Viral Protein Apoptin induces apoptosis in UV-C-irradiated cells from individuals with various hereditary cancer-prone syndromes. In press.
45. Zhuang S.-M., Landegent J.E., Verschueren C.A.J., Falkenburg J.H.F., Van Ormondt H., Van der Eb A.J., Noteborn M.H.M. Apoptin, a protein encoded by chicken anemia virus, induces cell death in various human hematologic malignant cells *in vitro.* (1995). Leukemia 9 S1, 118-120.
46. Zhuang S.-M., Shvarts A., Jochemsen A.-G., Van Oorschot A.A.A.M., Van der Eb A.J., Noteborn M.H.M. Differential sensitivity to Ad5 E1B-21kD and Bcl-2 proteins of apoptin-induced versus p53-induced apoptosis. (1995a). Carcinogenesis 16: 2939-2945.
47. Zhuang S.-M., Shvarts A., Van Ormondt H., Jochemsen A.-G., Van der Eb A.J., Noteborn M.H.M. Apoptin, a protein derived from chicken anemia virus, induces a p53-independent apoptosis in human osteosarcoma cells. (1995b). Cancer Research 55, 486-489.

## Claims

1. Use of an apoptosis inducing agent, which exhibit its effect in aberrant cells involved with or related to (auto) immune diseases, in the preparation of a medicament for the treatment of inflammatory disorders, wherein said apoptosis inducing agent is the apoptosis inducing protein apoptin or other proteins with apoptin-like activity.

2. Use of an apoptosis inducing agent, which exhibit its effect in aberrant cells involved with or related to (auto) immune diseases, in the preparation of a medicament for the treatment of immune diseases, wherein said apoptosis inducing agent is the apoptosis inducing protein apoptin or other proteins with apoptin-like activity.

3. Use of an apoptosis inducing agent according to claim 2 wherein said treatment of immune diseases is treatment of autoimmune diseases.

4. Use of a gene delivery vehicle comprising a gene capable of expressing an apoptosis inducing agent, which exhibit its effect in aberrant cells involved with or related to (auto) immune diseases, in the preparation of a medicament for the treatment of inflammatory disorders, wherein said apoptosis inducing agent is the apoptosis inducing protein apoptin or other proteins with apoptin-like activity.

5. Use of a gene delivery vehicle comprising a gene capable of expressing an apoptosis inducing agent, which exhibit its effect in aberrant cells involved with or related to (auto) immune diseases, in the preparation of a medicament for the treatment of immune diseases, wherein said apoptosis inducing agent is the apoptosis inducing protein apoptin or other proteins with apoptin-like activity.

6. Use of a gene delivery vehicle according to claim 5 wherein said treatment of immune diseases is treatment of autoimmune diseases.

7. Use according to anyone of claims 4-6, wherein said gene delivery vehicle further comprises a suicide gene.

8. Use according to claim 7, wherein said suicide gene is inducible.

9. Use according to anyone of claims 4-8, wherein said gene delivery vehicle has a tropism for hematopoietic cells.

10. Use according to claim 4-8, wherein said gene delivery vehicle has a tropism for fibroblast-like synoviocytes.

11. Use according to anyone of claims 4-10, wherein said gene delivery vehicle has been provided with a targeting means, especially a targetting means for fibroblast-like synoviocytes.

12. Use according to any one of claims 4-11, wherein said gene delivery vehicle comprises a recombinant adenovirus.

13. Use according to anyone of the aforegoing claims, wherein the apoptosis inducing agent comprises apoptin or a functional fragment, derivative or equivalent thereof.

14. Use according to anyone of the aforegoing claims wherein said apoptosis inducing agent is inducible.

15. A method for determining the presence of cells likely to result in an (auto)immune disease in a sample, comprising providing suspect cells with a protein having apoptin-like activity and subjecting said cells to stress, such as heat shock, osmotic shock, UV or chemical stress and determining apoptosis, wherein said protein having apoptin-like activity is provided by a nucleic acid molecule encoding said protein having apoptin-like activity.

16. A method for determining the presence of autoimmune diseases in an individual, comprising having provided a sample from said individual, said sample comprising cells implicated in said autoimmunedisease, providing said cells with a protein having apoptin-like activity and determining apoptosis, wherein said protein having apoptin-like activity is provided by a nucleic acid molecule encoding said protein having apoptin-like activity.

## Patentansprüche

1. Verwendung eines Apoptosis induzierenden Mittels, das seine Wirkung in aberranten Zellen zeigt, die an (Auto)Immunerkrankungen beteiligt sind oder mit diesen in Zusammenhang stehen, in der Zubereitung eines Medikaments zur Behandlung von entzündlichen Erkrankungen, wobei das Apoptosis induzierende Mittel das Apoptosis induzierende Protein Apoptin oder ein anderes Protein mit apoptinartiger Aktivität ist.

2. Verwendung eines Apoptosis induzierenden Mittels, das seine Wirkung in aberranten Zellen zeigt, die an (Auto)Immunerkrankungen beteiligt sind oder mit diesen in Zusammenhang stehen, in der Zubereitung eines Medikaments zur Behandlung von Immunerkrankungen, wobei das Apoptosis induzierende Mittel das Apoptosis induzierende Protein Apoptin oder ein anderes Protein mit apoptinartiger Aktivität ist.

3. Verwendung eines Apoptosis induzierenden Mittels nach Anspruch 2, wobei die Behandlung von Immunerkrankungen die Behandlung von Autoimmunerkrankungen ist.

4. Verwendung eines Genabgabevehikels, umfassend ein Gen, das imstande ist ein Apoptosis induzierendes Mittel zu exprimieren, das seine Wirkung in aberranten Zellen zeigt, die an (Auto)Immunerkrankungen beteiligt sind oder mit diesen in Zusammenhang stehen, in der Zubereitung eines Medikaments zur Behandlung von entzündlichen Erkrankungen, wobei das Apoptosis induzierende Mittel das Apoptosis induzierende Protein Apoptin oder ein anderes Protein mit apoptinartiger Aktivität ist.

5. Verwendung eines Genabgabevehikels, umfassend ein Gen, das imstande ist ein Apoptosis induzierendes Mittel zu exprimieren, das seine Wirkung in aberranten Zellen zeigt, die an (Auto)Immunerkrankungen beteiligt sind oder mit diesen in Zusammenhang stehen, in der Zubereitung eines Medikaments zur Behandlung Immunerkrankungen, wobei das Apoptosis induzierende Mittel das Apoptosis induzierende Protein Apoptin oder ein anderes Protein mit apoptinartiger Aktivität ist.

6. Verwendung eines Genabgabevehikels nach Anspruch 5, wobei die Behandlung von Immunerkrankungen die Behandlung von Autoimmunerkrankungen ist.

7. Verwendung nach einem der Ansprüche 4 bis 6, wobei das Genabgabevehikel des Weiteren ein Suizidgen umfasst.

8. Verwendung nach Anspruch 7, wobei das Suizidgen induzierbar ist.

9. Verwendung nach einem der Ansprüche 4 bis 8, wobei das Genabgabevehikel einen Tropismus für hämopoetische Zellen aufweist.

10. Verwendung nach einem der Ansprüche 4 bis 8, wobei das Genabgabevehikel einen Tropismus für fibroblastartige Synoviozyten aufweist.

11. Verwendung nach einem der Ansprüche 4 bis 10, wobei das Genabgabevehikel mit einem zielgerichteten Mittel versehen ist, insbesondere einem zielgerichteten Mittel für fibroblastartige Synoviozyten.

12. Verwendung nach einem der Ansprüche 4 bis 11, wobei das Genabgabevehikel ein rekombinantes Adenovirus umfasst.

13. Verwendung nach einem der vorhergehenden Ansprüche, wobei das Apoptosis induzierende Mittel Apoptin oder ein funktionales Fragment, Derivat oder Äquivalent davon umfasst.

14. Verwendung nach einem der vorhergehenden Ansprüche, wobei das Apoptosis induzierende Mittel induzierbar ist.

15. Verfahren zum Bestimmen der Gegenwart von Zellen, die wahrscheinlich zu einer (Auto)Immunerkrankung führen, in einer Probe, umfassend das Versehen verdächtiger Zellen mit einem Protein, das apoptinartige Aktivität besitzt, und Aussetzen der Zellen einer Belastung, wie einem Wärmeschock, osmotischem Schock, einer UV- oder chemischen Belastung, und Bestimmen der Apoptosis, wobei das Protein mit apoptinartiger Aktivität durch ein Nukleinsäuremolekül bereitgestellt wird, welches für das Protein mit apoptinartiger Aktivität codiert.

16. Verfahren zum Bestimmen des Vorliegens von Autoimmunerkrankungen in einem Individuum, umfassend das Erhalten einer Probe von dem Individuum, wobei die Probe Zellen umfasst, die an der Autoimmunerkrankung beteiligt sind, Versehen der Zellen mit einem Protein, das apoptinartige Aktivität besitzt, und Bestimmen der Apoptosis, wobei das Protein mit apoptinartiger Aktivität durch ein Nukleinsäuremolekül bereitgestellt wird, welches für das Protein mit apoptinartiger Aktivität codiert.

## Revendications

1. Utilisation d'un agent induisant l'apoptose qui montre ses effets dans des cellules aberrantes impliquées dans ou liées à des maladies (auto-immunes) dans la fabrication d'un médicament pour le traitement des désordres inflammatoires où ledit agent induisant l'apoptose est la protéine apoptine induisant l'apoptose ou d'autres protéines ayant une activité analogue à celle de l'apoptine.

2. Utilisation d'un agent induisant l'apoptose qui montre ses effets dans des cellules aberrantes impliquées dans ou liées à des maladies (auto-immunes) dans la fabrication d'un médicament pour le traitement des maladies immunes où ledit agent induisant l'apoptose est la protéine apoptine induisant l'apoptose ou d'autres protéines ayant une activité analogue à celle de l'apoptine.

3. Utilisation d'un agent induisant l'apoptose selon la revendication 2, dans laquelle ledit traitement des maladies immunes est le traitement des maladies auto-immunes.

4. Utilisation d'un véhicule de délivrance d'un gène comprenant un gène capable d'exprimer un agent induisant l'apoptose qui montre ses effets dans les cellules abeirantes impliquées dans ou liées à des maladies (auto-immunes) dans la fabrication d'un médicament pour le traitement des désordres inflammatoires ou ledit agent induisant l'apoptose et la protéine apoptine induisant l'apoptose ou d'autres protéines ayant une activité analogue à celle de l'apoptine.

5. Utilisation d'un véhicule de délivrance d'un gène comprenant un gène capable d'exprimer un agent induisant l'apoptose qui montre ses effets dans les cellules aberrantes impliquées dans ou liées à des maladies (auto-immunes) dans la fabrication d'un médicament pour le traitement de maladies immunes où ledit agent induisant l'apoptose est la protéine apoptine induisant l'apoptose ou d'autres protéines ayant une activité analogue à celle de l'apoptine.

6. Utilisation d'un véhicule de délivrance d'un gène selon la revendication 5, dans lequel ledit traitement des maladies immunes et le traitement des maladies auto-immunes.

7. Utilisation selon l'une quelconque des revendications 4-6, dans laquelle ledit véhicule de délivrance d'un gène comprend de plus un gène suicide.

8. Utilisation selon la revendication 7, dans laquelle ledit gène suicide est inductible.

9. Utilisation selon l'une quelconque des revendications 4-8, dans laquelle ledit véhicule de délivrance d'un gène a un tropisme vers les cellules hématopoïétiques.

10. Utilisation selon l'une quelconque des revendications 4-8, dans laquelle ledit véhicule de délivrance d'un gène a un tropisme pour des synoviocytes analogues aux fibroblastes.

11. Utilisation selon l'une quelconque des revendications 4-10, dans laquelle ledit véhicule de délivrance d'un gène a été fourni avec des moyens de ciblage, spécialement des moyens de ciblage vers les synoviocytes analogues aux fibroblastes.

12. Utilisation selon l'une quelconque des revendications 4-11, dans laquelle ledit véhicule de délivrance d'un gène comprend un adénovirus recombinant.

13. Utilisation selon l'une quelconque des revendications précédentes, dans laquelle l'agent induisant l'apoptose comprend l'apoptine ou un fragment fonctionnel, un dérivé ou un équivalent de celle-ci.

14. Utilisation selon l'une quelconque des revendications précédentes, dans laquelle ledit agent induisant l'apoptose est inductible.

15. Une méthode pour déterminer la présence de cellules résultant vraisemblablement d'une maladie (auto-immune) dans un échantillon comprenant la fourniture de cellules suspectes avec une protéine ayant une activité analogue à celle de l'apoptine et la soumission desdites cellules à un stress telles qu'un choc thermique, un choc osmotique, un choc chimique ou UV et la détermination de l'apoptose dans laquelle ladite protéine ayant une activité analogue à celle de l'apoptine est fournie par une molécule d'acide nucléique codant pour ladite protéine ayant une activité analogue à celle de l'apoptine.

16. Une méthode pour déterminer la présence de maladies auto-immunes chez un individu comprenant le fait d'avoir fourni un échantillon à partir dudit individu, ledit échantillon comprenant les cellules impliquées dans ladite maladie auto-immune, la fourniture desdites cellules avec une protéine ayant une activité analogue à celle de l'apoptine et la détermination de l'apoptose dans laquelle ladite protéine ayant une activité analogue à celle de l'apoptine et fourme par une molécule d'acide nucléique codant pour ladite protéine ayant une activité analogue à celle de l'apoptine.
